Europäisches Patentamt

European Patent Office

Office européen des brevets

(11) Publication number: **0 467 409 A1**

# EUROPEAN PATENT APPLICATION

(21) Application number: **91112194.5**

(22) Date of filing: **19.07.91**

(51) Int. Cl.⁵: **A61F 13/15**

(30) Priority: **20.07.90 US 556060**

(43) Date of publication of application:
**22.01.92 Bulletin 92/04**

(84) Designated Contracting States:
**BE DE DK FR GB LU NL**

(71) Applicant: **KIMBERLY-CLARK CORPORATION**
**401 North Lake Street**
**Neenah Wisconsin 54957-0349(US)**

(72) Inventor: **Kuen, David Arthur**
**1086 Honeysuckle Lane**
**Neenah, Wisconsin 54956(US)**
Inventor: **McFarland, Timothy Maurice**
**113 Law Street**
**Neenah, Wisconsin 54956(US)**

(74) Representative: **Patentanwälte Grünecker,**
**Kinkeldey, Stockmair & Partner**
**Maximilianstrasse 58**
**W-8000 München 22(DE)**

(54) **Absorbent pad having a reinforcing web.**

(57) A reinforced absorbent pad having a reinforcing web (16) located adjacent a layer of absorbent material. The reinforcing web (16) comprises intersecting strands, said strands being formed from a first polymeric material having a first melting point (20) and a second polymeric material having a second melting point (18) wherein said second melting point is lower than said first melting point. Thus, when the reinforcing web-containing absorbent pads are exposed to a temperature above the second melting point but below the first melting point the second polymeric material melt fuses to the surrounding fibers (14,12) while the first polymeric material (18) remains substantially intact, thus providing the absorbent pad with improved integrity. A method and apparatus of forming the described structures is also described.

FIG. 1

## Background of the Invention

### Field of the Invention

This invention relates to absorbent pads having a reinforcing web and to a method and apparatus for forming such reinforced absorbent pads.

### Description of the Related Art

Absorbent pads are used in items such as disposable diapers, adult incontinent products, sanitary napkins, wound dressings, and the like. As a general rule, such absorbent pads are maintained in a particular location with respect to a user, and are able to rapidly receive and absorb a discharged body fluid.

Absorbent pads, such as those described above, are frequently formed from fibrous material, preferably from cellulosic fibrous material, such as defibrated wood fibers commonly known as wood pulp fluff. One method of manufacturing absorbent pads from wood pulp fluff involves air laying the fibers on a porous substrate such as a vacuum drum to build up a pad having the desired thickness. When absorbent pads are formed from airlaid wood pulp fluff, the structural integrity of the absorbent pad results from the mechanical entanglement of fibers as they are airlaid on the substrate. As a general rule, such airlaid pads of fibers possess a low degree of structural integrity.

In use, such absorbent pads are often exposed to a variety of mechanical forces. For example, when such absorbent pads are employed in disposable diapers, the movement of the baby on which they are located places stress on the absorbent pad. The stresses are often sufficient to cause the absorbent pads to separate or clump in various locations in the diaper. Separation of the absorbent pad often causes a loss in the absorbent efficiency of the pad and in comfort to a user.

Various methods have been suggested to increase the integrity of absorbent pads formed from materials such as airlaid wood pulp fluff. For example, U.S. Patent 3,856,012 issued December 24, 1974 to MacDonald et al. is directed to a stabilized absorbent pad. The described pad comprises a plurality of elongated filaments which are incorporated into a wood pulp fluff pad. The filaments are spaced apart and each filament bears an adhesive which extends from the filament into the fibrous material of the pad to contact and bond individual fibers to the filament. U.S. Patent 3,768,479 issued October 30, 1973 to Widlund is directed to a disposable diaper. The form stability of the diaper is said to be good partly because the absorbent material in the diaper is secured by a binder and partly because an elastic insert in the form of a plastic mesh is placed inside said absorbent material. Similarly, U.S. Patent 3,587,579 issued July 28, 1971 to Sabee is directed to a reinforced diaper comprising a scrim made of woven or nonwoven fabric and incorporated into the absorbent pad of the diaper.

U.S. Patent 4,217,078 issued August 12, 1980 to Buell describes an apparatus for forming a reinforced fibrous web. The reinforcing web is described as being substantially impervious to the passage of fibers. In the described apparatus, a folding board is employed to maintain the major planar surface of the reinforcing web perpendicular to the fibrous web through a portion of the forming process.

The described reinforced absorbent pads nonetheless suffer from various drawbacks. For example, it is sometimes difficult to anchor the reinforcing web to the absorbent pad. Moreover, if this anchoring is done through the use of certain adhesive materials, the absorption capabilities of the absorbent pad may be deleteriously affected. Additionally, methods of producing reinforced absorbent pads are complex and inefficient.

## Summary of the Invention

It is desirable to produce an absorbent pad which is reinforced through the presence of a reinforcing web. It is further desired that the presence of said reinforcing web does not significantly deleteriously affect the absorption capabilities of the absorbent pad. Moreover, it is desired to provide a method and apparatus for incorporating the reinforcing web into the absorbent pad, which method and apparatus are simple and efficient. Further, it is desired that said method and apparatus allow the reinforcing web to be placed at various locations throughout the thickness (Z-direction) of the absorbent pad.

These and other related goals are achieved in an absorbent pad including a layer of absorbent material and a reinforcing web of material adjacent and contacting said layer of absorbent material. The reinforcing web comprises a network of intersecting strands and comprises a first polymeric material having a first melting point and a second polymeric material having a second melting point. When the absorbent pad is exposed to a temperature above the melting point of the second polymeric material, but below the melting point of the first polymeric material, the second polymeric material melt fuses to the layer of absorbent material, thereby anchoring the reinforcing web to the absorbent material while the first polymeric material enhances the structural integrity of the absorbent pad.

In one preferred embodiment, the absorbent material comprises two layers with the reinforcing web located between said layers; and the reinforcing web comprises a network of intersecting strands, which strands have an inner core formed from the first polymeric material and an outer sheath formed from the second polymeric material. Again, after the reinforcing web has been located between the two layers of absorbent material, the absorbent pad can be exposed to a temperature above the melting point of the second polymeric material but below the melting point of the first polymeric material. In this manner, the second polymeric material melt fuses to at least one of the layers of absorbent material, thus anchoring the reinforcing web to the absorbent material, while the first polymeric material remains intact to enhance the structural integrity of the absorbent pad.

In another aspect, the present invention concerns a method for forming a reinforced web of fibers, said method involving the steps of introducing a reinforcing web of material into a forming chamber, passing air-suspended fibers present in said forming chamber through said reinforcing web and onto a porous surface to form a first layer of fibers, laying said reinforcing web on said first layer of fibers and forming a second layer of fibers on said reinforcing web.

In yet another aspect, the present invention concerns an apparatus for accomplishing the method described above and forming the absorbent pad described above. The apparatus comprises a forming chamber for containing air suspended fibers, vacuum deposition means in operable relation to said forming chamber for receiving deposited fibers, means for supplying a reinforcing web of material to said forming chamber, and an adjustable means for controlling the point at which the reinforcing web contacts the deposited fibers in the forming chamber.

## Brief Description of the Drawings

Figure 1 is a perspective view of an absorbent pad according to one embodiment of the present invention.

Figure 2 is a partial, enlarged, cross-sectional view taken along line 2-2 of Figure 1 prior to exposing the pad to an elevated temperature.

Figure 3 is a partial, enlarged, cross-sectional view taken along line 2-2 of Figure 1 after exposing the pad to an elevated temperature.

Figure 4 is a perspective view of the embossed absorbent pad illustrated in Figure 1 after being embossed at an elevated temperature and pressure.

Figure 5 is a partial, enlarged, cross-sectional view taken along line 5-5 of Figure 4.

Figures 6 and 7 are schematic illustrations of the apparatus according to the present invention.

## Detailed Description of the Preferred Embodiment

The present invention relates to an absorbent pad including a layer of absorbent material and a reinforcing web of material adjacent and contacting said layer of absorbent material. The reinforcing web comprises a network of intersecting strands and comprises a first polymeric material having a first melting point and a second polymeric material having a second melting point. The second melting point is lower than the first melting point. It is to be understood that reference to an absorbent pad is intended to encompass any fibrous mat of material. Preferred embodiments of the present invention can best be understood by reference to the accompanying drawings.

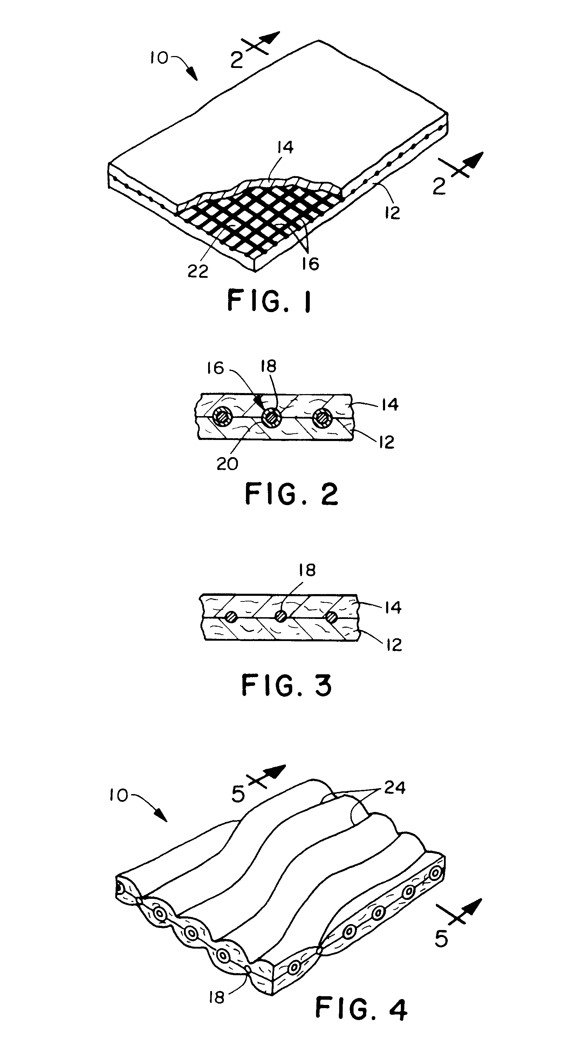

With reference to Figure 1, a preferred reinforced absorbent pad according to the present invention is represented generally by the number 10. The reinforced absorbent pad 10 comprises a first layer of absorbent material 12, a second layer of absorbent material 14 and a reinforcing web 16 located between first layer 12 and second layer 14. The first and second layers of absorbent material are formed from fibers which have been formed into pads. Exemplary of fibers from which the first and second layers of absorbent material can be formed are cellulosic fibers such as wood pulp fluff, cotton, regenerated cellulosic materials such as rayon, synthetic fibers such as polyester and nylon fibers, and the like. The fibers may also comprise a mixture of cellulosic fibers and synthetic polymer fibers. For example, the material may comprise a blend of cellulosic fibers such as wood pulp fluff and meltblown polyolefin fibers, such as polyethylene and polypropylene fibers.

Methods of forming the layer of absorbent material are known to those skilled in the art. Typically, the fibers will be incorporated into a stream of air which deposits the fibers on a porous forming surface such as a vacuum drum. Methods of forming the absorbent pads of the present invention will be discussed in greater detail below.

The layer of absorbent material suitably has a density within the range of from about 0.04 to about 0.3 grams per cubic centimeter, preferably from about 0.08 to about 0.20 grams per cubic centimeter measured under a restraining load of 0.1 pounds per square inch.

The reinforcing web 16 comprises a network of intersecting strands, said strands preferably being bonded at the points of intersection. The web comprises at least two different polymeric materials. Specifically, the web comprises a first polymeric material having a first melting point and a second polymeric material having a second melting point. The melting point of the second polymeric material is less than the melting point of the first polymeric material.

In one preferred embodiment, the individual strands from which the reinforcing web is formed have an inner core formed from the first polymeric material having a first melting point and an outer sheath formed from the second polymeric material having a second melting point wherein the second melting point is lower than the first melting point. Alternatively, the web may be formed from strands of the first polymeric material in combination with strands formed from the second polymeric material.

Figure 2 is a partial enlarged cross-sectional view taken along line 2-2 of Figure 1. As can be seen from reference to Figure 2, reinforcing web 16 comprises, in a preferred embodiment, individual strands having an inner core 18 and an outer sheath 20. The individual strands of reinforcing web 16 may be formed by any method suitable to produce the described structure. Suitably, the inner core and sheath are coextruded in a melt extrusion process. Such processes are known to those skilled in the art. The outer sheath 20 may be generally continuous around the inner core 18 or may be discontinuous in certain locations. It is preferred that the sheath be generally continuous around the inner core 18.

Additionally, while it is preferred that all of the strands of reinforcing core 16 have the described inner core/outer sheath construction, it is possible to form a web comprising a combination of strands formed from the first polymeric material and strands formed from the second polymeric material. For example, every other strand in both the transverse and longitudinal direction of the web may be formed from the first polymeric material with the alternate strands being formed from the second polymeric material. Clearly, a wide variety of combinations are possible. It is generally preferred that the ratio of first polymeric material to second polymeric material present in the web be within the range of from about 90:10 to about 10:90 more preferably within the range of from about 65:35 to about 35:65.

When the reinforcing web comprises strands having the described inner core/outer sheath construction, it is generally possible to form up to about 50 percent of the strands in a non-inner core/outer sheath construction. For example, up to about 50 percent of the strands may comprise a single strand of a single polymeric material with at least about 50 percent of the strands having the inner core/outer sheath construction. Of the strands having the inner core/outer sheath construction, it is generally preferred that the strands be present in both the longitudinal and transverse direction in the reinforcing web. However, it is possible to form a reinforcing web where only the strands in either the longitudinal or transverse direction have the inner core/outer sheath construction.

The first polymeric material and second polymeric material can be selected from a wide variety of polymeric materials, so long as the first polymeric material has a melting point which is higher than the melting point of the second polymeric material. It is generally preferred that the difference between the melting point of the first polymeric material and the melting point of the second polymeric material be at least about 5 degrees Centigrade, preferably at least about 20 degrees Centigrade. Exemplary of materials suitable for use as the first or second polymeric material are polypropylene, polyethylene, polyesters, ethylene vinyl acetate, nylon-6, nylon-66 and the like. Those skilled in the art will be able to select suitable combinations of polymeric materials for use in the present invention. In one preferred embodiment of the present invention, the first polymeric material is polypropylene and the second polymeric material is polyethylene. It is further preferred that the strands have the described inner core/outer sheath construction.

The strands forming the reinforcing web 16 may comprise a wide variety of cross sectional shapes. In the embodiment illustrated in Figures 1-5, the strands are indicated as having a generally circular cross sectional shape and as having the inner core/outer sheath construction. Generally, the strands will have a size and shape such that the flexibility of the layer of absorbent material is not significantly, deleteriously affected. When the strands have a generally circular cross section, the strands will suitably have a diameter within the range of from about 0.02 to about 1.0 millimeter, preferably within the range of from about 0.04 to about 0.25 millimeter. Moreover, when the strands have a diameter within the range of from about 0.04 to about 0.25 millimeter, and have the inner core/outer sheath construction, the diameter of the inner core 18 will suitably be within the range of from about 0.02 to about 0.24 millimeter. In any event, the inner core must be of sufficient size to provide the desired degree of structural integrity.

Methods of forming coextruded plastic nets comprising strands having an inner core/outer sheath configuration are generally described in U.S. Patent 4,410,587 issued October 18, 1983 to Fair et al. and U.S. Patent 4,656,075 issued April 7, 1987 to Mudge, which patents are hereby incorporated by reference. Methods of forming webs comprising strands of the first polymeric material and the second polymeric material are known to those skilled in the art.

The intersecting strands of the reinforcing web 16 define openings 22. As will become more apparent in connection with the method and apparatus described below, the openings 22 defined by the strands of the reinforcing web 16 must be large enough to allow a desired amount of fibrous material from which the first layer of absorbent material is formed to pass therethrough during the formation process. Accordingly, the openings 22 suitably have an area of from about 9 to about 900, preferably from about 25 to about 400 square millimeters. In the preferred embodiment the openings are square or rectangular in configuration.

When the absorbent pads according to the present invention are exposed to a temperature above the melting point of the second polymeric material but below the melting point of the first polymeric material, the second polymeric material softens and eventually melts, thus bonding the fibers in the vicinity of the second polymeric material together upon resolidification of the second polymer as a result of subsequent cooling to a temperature below said second melting point. In the illustrated embodiment, outer sheath 20 softens or melts while inner core 18 remains intact. In this manner, structural integrity is imparted to the first and second layers of absorbent material. Figures 1 and 2 illustrate an absorbent pad according to the present invention prior to being subjected to a heat treatment. Figure 3 is the cross section illustrated in Figure 2 after exposure to a heat treatment sufficient to cause the outer sheath 20 to melt. Thus, all that remains as an integral structure is inner core 18.

The heat treatment may be effected by raising the entire absorbent pad to the desired temperature. Alternatively, selected portions of the absorbent pad may be subjected to the heat treatment while other portions are not subjected to the heat treatment. Additionally, it may be desirable to subject the heated portions of the pad to an elevated pressure to assist in distributing the softened outer sheath material to neighboring fibers. For example, absorbent pads such as that illustrated in Figure 1 may be embossed under temperature and pressure conditions which cause the outer sheath to melt and fuse to the surrounding material which allows the inner core to remain intact.

Figure 4 illustrates the absorbent pad illustrated in Figure 1 after having been embossed at a temperature and pressure sufficient to cause the outer sheath to melt and the inner core to remain intact. The absorbent pad 10 has been embossed along lines 24. Those skilled in the art will recognize suitable means for embossing the absorbent pad 10 at the required temperature and pressure.

In the preferred embodiment discussed above, wherein the inner core is formed from polypropylene and the outer sheath is formed from polyethylene, the absorbent pad is suitably heated to a temperature within the range of from about 110°C to about 155°C to melt the outer sheath. When the heating is accomplished through an embossing process, the embossing occurs under a pressure within the range of from about 400 pounds per effective lineal inch to about 2000 pounds per effective lineal inch. When pressure is applied to the absorbent pad during the heating process, the temperature to which the pad is heated is generally lower than when the heating occurs in the absence of pressure.

The layer of absorbent material may comprise an effective amount of an inorganic or organic high-absorbency material to enhance the absorptive capacity of the pad. For example, the absorbent layer can include 5-95 weight percent of a high-absorbency material and preferably includes from about 10 to about 50 weight percent of the high absorbency material to provide a more efficient performance. Suitable inorganic high-absorbency materials include, for example, absorbent clays and silica gels. Organic high-absorbency materials can include natural materials such as agar, pectin, guar gum, and peat moss, as well as synthetic materials such as synthetic hydrogel polymers. Such hydrogel polymers include, for example, carboxymethyl cellulose, alkali metal salts of polyacrylic acids, polyacrylamides, polyvinyl alcohol, ethylene maleic anhydride copolymers, polyvinyl ethers, hydroxypropylcellulose, polyvinyl morpholinone, polymers and copolymers of vinyl sulfonic acid, polyacrylates, polyacrylamides, polyvinylpyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly cross-linked to render the materials substantially water-insoluble.

The high-absorbency material can be distributed or otherwise incorporated into one or both of the first or second layers of absorbent material. For example, the high-absorbency material can be mixed with the fibers during formation of the first and/or second layers of absorbent material.

When one or both of the first or second layers of absorbent material comprise a high-absorbency material in particulate form, it is often desirable to apply a tissue wrap around the absorbent pad. The tissue wrap suitably has a basis weight of less than about 40 grams per square meter and aids in containing the high-absorbency material. Tissue wraps suitable for use in the present invention are known to those skilled in the art.

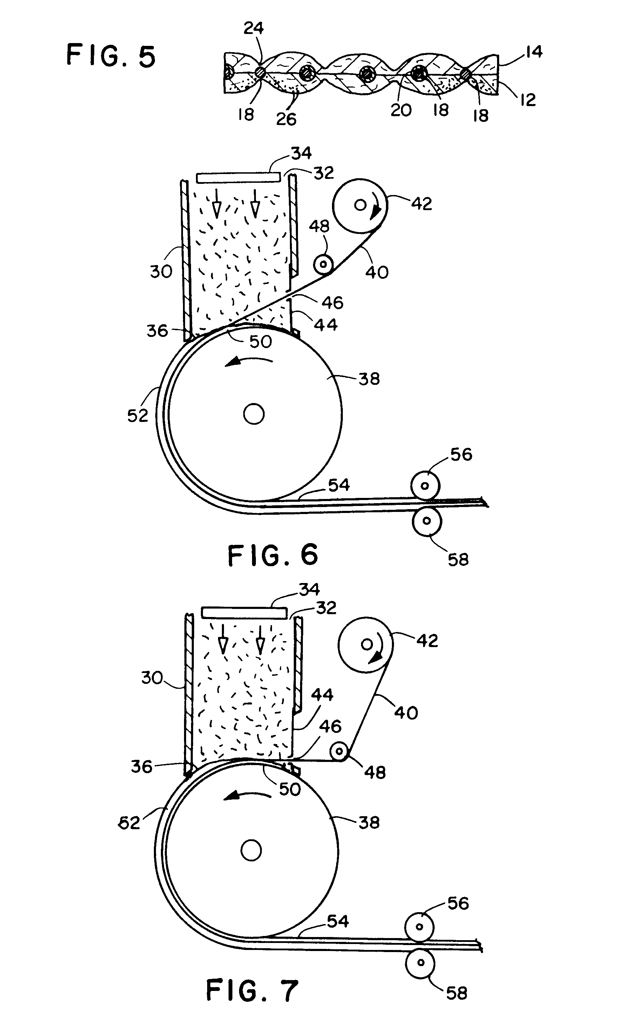

Figure 5 illustrates a cross section taken along line 5-5 of Figure 4 further including particles 26 of a high absorbency material. As can be seen from reference to Figure 5, when the absorbent pads of the present invention are exposed to an embossing process, not all of the strands of the reinforcing web 16 are caused to have the outer sheath 20 melted. Instead, only those strands at or near the location of embossing lines 24 are caused to have the outer sheath melt.

The absorbent pads according to the present invention suitably have a thickness of from about 1 to about 30 millimeters, preferably from about 2 to about 20 millimeters, and a density of from about 0.04 to about 0.3 grams per cubic centimeter, preferably from about 0.06 to about 0.2 grams per cubic centimeter when measured under a restraining load of 0.1 pounds per square inch.

The reinforcing web may be coextensive with the layers of absorbent material. Alternatively, for reasons such as cost, the reinforcing web may extend through only a portion of the absorbent pad. For example, the reinforcing web may, in one embodiment, extend along the entire length of the absorbent pad, but may be narrower than the absorbent pad. Clearly many variations are possible.

In another aspect, the present invention concerns a method for forming the preferred reinforced absorbent pad described above. The steps of the method include introducing a reinforcing web of material into a forming chamber. The reinforcing web may be a web such as those described above or may be a more conventional web formed from intersecting strands of a single polymeric material. Forming chambers are known to those skilled in the art and generally comprise a substantially enclosed area having, generally at one end thereof, means for providing a stream of air and fibrous material and, at the opposite end of the forming chamber, a porous deposition means. According to the method of the present invention, air suspended fibers present in said forming chamber are passed through the reinforcing web onto a deposition surface to form a first layer of fibers. The deposition means generally comprises a porous surface, which collects the fibers but allows air to pass therethrough. Such surfaces are known to those skilled in the art. Exemplary of such surfaces are screens, vacuum drums, perforated plates, and the like.

The reinforcing web of material is then laid on the first layer of fibers while said reinforcing web and first layer of fibers remains in the forming chamber. A second layer of fibers is then formed on top of the reinforcing web. The absorbent pad comprising the first and second layers of fibers and reinforcing web then exits the forming chamber. According to the method of the present invention, the location of the reinforcing web in the thickness of the absorbent pad (Z-direction) can be controlled by controlling the point at which the reinforcing web contacts the first layer of fibers. For example, assuming constant fiber deposition rates, if the reinforcing web contacts the first layer of fibers relatively quickly after entering the forming chamber, the first layer of fibers will be relatively thin with the second layer of fibers being relatively thick. Conversely, if the reinforcing web contacts the first layer of fibers relatively late after entering the forming chamber, the first layer of fibers will be relatively thick and the second layer of fibers will be relatively thin.

In a final aspect, the present invention concerns an apparatus for forming a reinforced absorbent pad. Such apparatus is illustrated in Figures 6 and 7. The apparatus illustrated in Figure 6 comprises an enclosed forming chamber 30, shown in cross section, for containing air suspended fibers. At end 32 of forming chamber 30 is means 34 for providing fibers suspended in air to forming chamber 30. Means 34 for providing air suspended fibers to forming chambers 30 are known to those skilled in the art and suitably comprise hammer mills, attrition mills, picker rolls, and the like. As discussed above, the fibers may comprise cellulosic fibers such as wood pulp fluff, synthetic or polymeric fibers, or a combination of cellulosic fibers and polymeric fibers. End 36 of forming chamber 30 is located opposite end 32. A deposition means, vacuum drum 38, for receiving deposited fibers from forming chamber 30, is located in an operable relationship with forming chamber 30. Other suitable means for receiving deposited fibers are known to those skilled in the art and may, for example, include screens, porous belts or the like.

Reinforcing web 40 is supplied to the forming chamber 30 from supply roll 42. The reinforcing web 40 enters the forming chamber 30 through an adjustable window means 44 which defines opening 46. The opening 46 is adjustable to different positions along adjustable window means 44. Positioning roll 48 assists in directing reinforcing web 40 into opening 46. Positioning roll 48, opening 46, and adjustable window means 44 cooperate to determine the point at which reinforcing web 40 contacts the first layer 50 of fibers deposited on vacuum roll 38.

As can be seen from reference to Figure 5, at the point at which reinforcing web 40 enters forming chamber 30, fibers present in the forming chamber begin to pass through the reinforcing web and are deposited on vacuum drum 38. The amount of fibers deposited, and thus the thickness of the deposited fibers, increases as the vacuum drum 38 rotates through forming chamber 30. At some point in the forming chamber 30, reinforcing web 40 contacts the first layer of deposited fibers 50. After this point of contact, fibers subsequently deposited on top of reinforcing web 40 constitute the second layer of deposited fibers 52.

After exiting forming chamber 30, the first and second layers of deposited fibers containing reinforcing web 40 continues around vacuum drum 38 until being removed therefrom at point 54. At this point, the first and second layers of deposited fibers and reinforcing web are passed through embossing roll 56 and anvil roll 58. Embossing roll 56 and anvil roll 58 cooperate to emboss the first and second layer of deposited fibers and reinforcing web 40. When reinforcing web 40 comprises a web as described above having an inner core/outer sheath construction, embossing roll 56 is suitably maintained at a temperature sufficient to cause the outer sheath to melt and fuse to the surrounding fibers.

The manner in which positioning roll 48, adjustable window means 44, and opening 46 cooperate to control the point at which reinforcing web 40 contacts the first layer of deposited fibers 50, can be seen by reference to Figure 7, when compared to Figure 6. Figure 7 is identical to Figure 6 except that the location of positioning roll 48 and opening 46 have been varied to cause reinforcing web 40 to contact the first layer of deposited fibers 50 relatively quickly after reinforcing web 40 enters forming chamber 30. Thus, while the total thickness of the deposited fibers and reinforcing web is the same in both Figures 6 and 7, reinforcing web 40 is located at different locations in the thickness (Z-direction) of said deposited fibers due to changing the position of positioning roll 48 and opening 46.

The reinforced absorbent pads described above are suitably incorporated as the absorbent structure in absorbent garments such as disposable diapers, adult incontinent care products, feminine napkins, tampons, and the like. As a general rule, such disposable garments comprise a fluid-pervious body side liner, a fluid-impervious outer cover, and an absorbent structure such as the absorbent pads described above, sandwiched between said fluid-pervious body side liner and said fluid-impervious outer cover. Absorbent garments into which the absorbent pads according to the present invention can be incorporated are described in U.S. Patent 4,798,603 issued January 17, 1989 to Meyer et al., 4,710,187 issued December 1, 1987 to Boland et al., 4,770,656 issued September 13, 1988 to Proxmire et al., and 4,762,521 issued August 9, 1988 to Roessler et al., which patents are hereby incorporated by reference.

Those skilled in the art will recognize that the present invention is capable of many modifications and variations without departing from the scope thereof. Accordingly, the detailed descriptions set forth above are meant to be illustrative only and are not meant to limit, in any manner, the scope of the invention as set forth in the following claims.

## Claims

1. An absorbent pad, said absorbent pad comprising:
   a layer of absorbent material; and
   a reinforcing web of material adjacent to and contacting said layer of absorbent material, said reinforcing web comprising a network of intersecting strands, said strands being formed from a first polymeric material having a first melting point and a second polymeric material having a second melting point, said second melting point being lower than said first melting point, wherein when said absorbent pad is exposed to a temperature above said second melting point but below said first melting point, said second polymeric material melt fuses to at least one of the first or second layers of absorbent material and said first polymeric material remains substantially intact.

2. The absorbent pad according to claim 1 wherein said layer of absorbent material comprises airlaid cellulosic fibers.

3. The absorbent pad according to claim 2 wherein said layer of absorbent material comprises a high-absorbency material.

4. The absorbent pad according to claim 3 wherein said cellulosic fibers are wood pulp fluff.

5. The absorbent pad according to claim 1 wherein said second melting point is at least about 5 degrees Centigrade lower than said first melting point.

6. The absorbent pad according to claim 1 wherein said strands have an inner core/outer sheath construction wherein said inner core is formed from said first polymeric material and said outer sheath is formed from said second polymeric material.

7. The absorbent pad according to claim 6 wherein said inner core is formed from polypropylene and said outer sheath is formed from polyethylene.

8. The absorbent pad according to claim 6 wherein said core is generally completely surrounded by said sheath.

9. The absorbent pad according to claim 1 wherein said reinforcing web comprises strands formed from said first polymeric material and strands formed from said second polymeric material in combination.

10. The absorbent pad according to claim 1 wherein said intersecting strands are adhered to one another at their point of intersection.

11. The absorbent pad according to claim 1 wherein said strands have a generally circular cross section, said cross section having a diameter of from about 0.02 to about 1.0 millimeter.

12. The absorbent pad according to claim 6 wherein said strands have a generally circular cross section, said cross section having a diameter of from about 0.04 to about 0.25 millimeter and wherein said inner core has a diameter of from about 0.02 to about 0.24 millimeter.

13. The absorbent pad according to claim 1 wherein said reinforcing web extends along the entire length of said absorbent pad.

14. The absorbent pad according to claim 13 wherein the reinforcing web has a width which is less than the width of said absorbent pad.

15. The absorbent pad according to claim 1 wherein the absorbent pad is embossed at a temperature above said second melting point and below said first melting point.

16. An absorbent pad, said absorbent pad comprising:
   a first layer of cellulosic fiber material;
   a second layer of cellulosic fiber material; and
   a reinforcing web of material located between said first and second layers of cellulosic fiber, said reinforcing web comprising a network of intersecting strands, said strands having an inner core formed from polypropylene and an outer sheath formed from polyethylene wherein, when said absorbent pad is exposed to a temperature above the melting point of polyethylene but below the melting point of polypropylene, the polyethylene outer sheath melt fuses to at least one of the first or second layers of cellulosic fiber material and the polypropylene core remains substantially intact.

17. An absorbent garment, said absorbent garment comprising:
   a water-impervious outer cover;
   a water-pervious body side liner;
   an absorbent pad located between said outer cover and said body side liner, said absorbent pad comprising:
   a first layer of absorbent material;

a second layer of absorbent material; and
   a reinforcing web of material located between said first and second layers of absorbent material, said reinforcing web comprising a network of intersecting strands, said strands being formed from a first polymeric material having a first melting point and a second polymeric material having a second melting point, said second melting point being lower than said first melting point wherein, when said absorbent pad is exposed to a temperature above said second melting point but below said first melting point, said second polymeric material melt fuses to at least one of the first or second layers of absorbent material and said first polymeric material remains substantially intact.

18. A method for forming a reinforced web of fibers, said method comprising the steps of:
   introducing a reinforcing web material into a forming chamber containing air suspended fibers;
   passing air suspended fibers present in said forming chamber through said reinforcing web and onto a deposition surface to form a first layer of deposited fibers;
   laying said reinforcing web on said first layer of deposited fibers; and
   forming a second layer of deposited fibers on said reinforcing web.

19. The method according to claim 18 wherein said reinforcing web of material comprises a network of intersecting strands.

20. The method according to claim 19 wherein said strands have an inner core formed from a first polymeric material having a first melting point and an outer sheath formed from a second polymeric material having a second melting point, said second melting point being lower than said first melting point.

21. The method according to claim 20 further comprising the step of embossing said reinforced web of fibers at a temperature above the melting point of said second polymeric material and below the melting point of said first polymeric material.

22. An apparatus for forming a reinforced web of fibers, said apparatus comprising:
   a forming chamber for containing air suspended fibers;
   deposition means in operable relation to said forming chamber for receiving deposited fibers;

means for supplying a reinforcing web of material to said forming chamber; and

adjustable means for controlling the point at which said reinforcing web contacts fibers deposited on said deposition means.

23. The apparatus according to claim 22 wherein said deposition means comprises a vacuum drum.

24. The apparatus according to claim 22 wherein said means for supplying a reinforcing web of material to said forming chamber comprises a supply roll.

25. The apparatus according to claim 22 wherein said adjustable means for controlling the point at which said reinforcing web contacts fibers deposited on said deposition means comprises a positioning roll and an adjustable window means.

FIG. 1

FIG. 2

FIG. 3

FIG. 4

FIG. 5

FIG. 6

FIG. 7

European
Patent Office

EUROPEAN SEARCH
REPORT

Application Number

EP 91 11 2194

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 544 596   (PERSONAL PRODUCTS COMPANY) * column 5, line 32 - line 49 * * * column 6, line 47 - line 63; figures 5,6 * * | 1,2,4-6,8, 10-14,16, 17 | A 61 F 13/15 |
| Y | | 9,19-25 | |
| | – – – | | |
| X | US-A-4 237 591   (PERSONAL PRODUCTS COMPANY) | 18 | |
| | – – – | | |
| Y | US-A-4 237 591   (* column 5, line 31 - line 36; figures 5,6 *) | 19-25 | |
| | – – – | | |
| Y | FR-A-2 195 555   (BEGHIN-SAY) * claims 1,2,10,11; figures * * | 9 | |
| | – – – | | |
| X | EP-A-0 297 411   (PEAUDOUCE) * abstract * * * column 5, line 2 - line 53 * * | 1,3,5-8, 10,15 | |
| | – – – | | |
| A | US-A-4 519 799   (KAO SOAP CO. LTD) * the whole document * * | 1 | |
| | – – – – – | | |

TECHNICAL FIELDS
SEARCHED (Int. Cl.5)

A 61 F

The present search report has been drawn up for all claims

| Place of search | Date of completion of search | Examiner |
|---|---|---|
| The Hague | 15 November 91 | ARGENTINI A. |